Europäisches Patentamt

European Patent Office

Office européen des brevets

(11) Veröffentlichungsnummer: **0 374 915**
A2

## EUROPÄISCHE PATENTANMELDUNG

(21) Anmeldenummer: 89123655.6

(22) Anmeldetag: 21.12.89

(51) Int. Cl.5: **C12N 9/08, //C12N9/96**

(30) Priorität: 23.12.88 DE 3843381

(43) Veröffentlichungstag der Anmeldung:
27.06.90 Patentblatt 90/26

(84) Benannte Vertragsstaaten:
BE CH GB LI LU NL SE

(71) Anmelder: Forschungszentrum Jülich GmbH
Postfach 1913
D-5170 Jülich(DE)

(72) Erfinder: Kern, Hartmut, Prof. Dr.
Lövenicher Strasse 29
D-5172 Linnich(DE)

(54) **Verfahren zur Gewinnung ligninolytischer Enzyme.**

(57) Die Gewinnung ligninolytischer Enzyme durch Kultivierung von Weißfäulepilzen kann dadurch verbessert werden, daß der Pilzkultur bei beginnender Verfärbung zumindest 0,1 g/l, insbesondere 0,3 bis 5 g/l und speziell um 1 g/l Mangandioxid-Pulver, insbesondere feinteiliges Pulver mit einer Korngröße < 10 µm zugesetzt und der pH-Wert im Medium während der gesamten Kulturdauer zwischen 4,8 und 5,0 konstant gehalten wird. Vorzugsweise werden die Enzyme vom Kulturmedium wiederkehrend durch Ultrafiltration abgesondert und das Restkultursubstrat recycliert.

EP 0 374 915 A2

## Verfahren zur Gewinnung ligninolytischer Enzyme

Die Erfindung bezieht sich auf ein Verfahren zur Gewinnung ligninolytischer Enzyme durch Kultivierung von Weißfäulepilzen.

Ligninolytische Enzyme, die sog. Ligninasen sind Schlüsselenzyme beim biologischen Abbau von Lignin und Lignocellulosen. Sie dürften daher in der Zukunft eine beachtliche Rolle in der Praxis der Holz- und Papierindustrie, beim sog. Biopulping, für die Abwasserreinigung etc. spielen. Verfahren, welche die Produktion großer Mengen dieser Enzyme ermöglichen, sind daher von großem Interesse.

Ziel der Erfindung ist daher ein Verfahren, mit dem die Bildung solcher Ligninasen erheblich gefördert wird.

Das zu diesem Zweck entwickelte erfindungsgemäße Verfahren ist dadurch gekennzeichnet, daß der Pilz-Kultur bei beginnender Verfärbung zumindest 0,1 g/l Mangandioxid-Pulver zugesetzt und der pH-Wert im Medium während der gesamten Kulturdauer zwischen pH 4,8 und 5,0 konstant gehalten wird.

Es wurde überraschenderweise gefunden, daß der Zusatz von Mangandioxid zur Pilz-Kultur zu einer deutlichen Steigerung der gewinnbaren ligninolytischen Enzyme führt. Dabei scheint sowohl eine gesteigerte Produktion der Enzyme als auch ihre Stabilisierung gegenüber inaktivierenden Einflüssen der Kulturflüssigkeit gegeben zu sein: das von den Pilzen gleichzeitig mit besagten Enzymen gebildete Wasserstoffperoxid, durch das die Enzyme inaktiviert werden können, wird durch die Zugabe des Mangandioxids zur Pilz-Kultur katalytisch zersetzt, an deren Zellwände sich das $MnO_2$ anlagert, so daß das Pilz-Mycel nach der Zugabe schwarz erscheint. Das so behandelte Mycel ist für die Ligninase-Produktion über längere Zeit brauchbar, indem Kulturflüssigkeit abgetrennt und nach Gewinnung der Ligninasen durch Ultrafiltration ggf. unter Ergänzung der Substrate recycliert wird.

Das Mangandioxid wird in feinteiliger Form, insbesondere mit einer Korngröße < 10 $\mu$m zugesetzt, vorzugsweise in Mengen von 0,3 bis 5 g/l und speziell um etwa 1 g/l Kulturmedium. Benutzt wurde für die Untersuchungen im Handel erhältliches Mangan(IV)-oxidpulver (zerriebener Pyrolusit mit einer mittleren Korngröße von 7 $\mu$m). In gleicher Weise kann jedoch auch jede andere feinteilige Form von Mangandioxid (außer totgebranntem Oxid) angewandt werden.

Die Pilze können in sog. Schüttelkulturen als Mycelbällchen oder Pellets oder zusammenhängende Mycelmatten vorliegen oder auch auf Träger aufgezogen sein.

Alle bislang untersuchten Stämme von P. chrysosporium haben sich als tauglich erwiesen, wobei in den meisten Fällen eine Steigerung bis um den Faktor 2 der Enzymproduktion durch die Zugabe des Mangandioxids bewirkt werden konnte. Erfindungsgemäß kommt es durch die stabilisierende Wirkung des Mangandioxids nicht zu der sonst üblicherweise beobachteten Absenkung der Enzymaktivität, so daß die Wirtschaftlichkeit der Ligninasegewinnung auf diese Weise erheblich verbessert werden kann.

Das mit Mangandioxid behaftete Pilz-Mycel kann über längere Zeit bei Kühlschranktemperaturen (+4° C) zwischengelagert werden.

Es folgt ein Beispiel zur Erläuterung der Erfindung.

Beispiel:

Kultiviert wurden die Stämme ATCC 32629, Novobranova; ATCC 24725 und Stamm SC26, eine Mutante des Stamms ATCC 24725.

Nach einer Vorkultur in einem Glucose-Medium (2 Tage bei 39° C unter Luft in Fernbachkolben) wurde das gebildete Mycel mit destilliertem Wasser gewaschen und unter sterilen Bedingungen in destilliertem Wasser homogenisiert. Aliquote Teile dieses Homogenates wurden als Inokulum für die Versuchsansätze verwendet. In Anlehnung an bewährte und publizierte Medien (siehe T.K. Kirk u.a., Enzyme Microb. Technol. 8 (1986) 27-32 und H.W. Kern u.a., Appl. Environ.Microbiol. 53 (1987), 2242-46) wurde ein Kulturmedium der folgenden Zusammensetzung (N-Mangel) benutzt (pro l):

$K_2HPO_4$ 4,59 mmol; $KH_2PO_4$ 8,8 mmol; $MgSO_4$ 2 mmol; $CaCl_2$ 0,68 mmol; Thiamin 2,97 $\mu$mol; Ammoniumtartrat 1,1 mmol; Spurenelemente 10 ml einer Stammlösung nach T.K.Kirk (s. o.); Veratrylalkohol 0,4 mmol; Tween 80 0,5 g.

Als C-Quelle wurden Glucose oder Glycerin, beide je 10 g/l, verwendet. Der pH-Wert der Kulturflüssigkeit wurde mittels KOH eingestellt. Eine zusätzliche Pufferung des Mediums während der Kultivierung wurde durch Zusatz von cis,trans-Aconitsäure (6 mmol/l) vorgenommen.

Die Kultivierung des Pilzes erfolgte in unterschiedlich großen Erlenmeyerkolben (200 ml Kolben mit 70 ml bzw. 1 l Kolben mit 300 ml des beimpften Mediums). Nach Begasen der Kolben mit Sauerstoff wurden diese mit Silikonstopfen verschlossen und bei 39° C und einer Schüttelfrequenz von 130 RpM (Pilot-Shaker, Fa. Braun) inkubiert.

Nach ca. 2 d Inkubation zeigten die zu Kugeln (sog. Pellets) herangewachsenen Pilz-Mycelien eine beginnende Verfärbung (dunkel-braun). Zu diesem Zeitpunkt wurden die Kulturen mit festem, sterilisiertem Mangandioxid ($MnO_2$) versetzt (1 g $MnO_2$ l), erneut mit Sauerstoff begast und unter sonst gleichen Bedingungen weiterkultiviert.

Der drastische Unterschied in der Ligninaseproduktion von Kulturen, die ohne oder mit Zusatz von $MnO_2$ erfolgten, ergibt sich aus der folgenden Tabelle:

| Kulturdauer | 3 d | 4 d | 5 d |
|---|---|---|---|
| Ligninase in U/l [*] ohne $MnO_2$ | 301 | 570 | 410 |
| mit $MnO_2$ | 309 | 1.125 | 1.304 |

[*] Die Bestimmung der enzymatischen Aktivitäten erfolgte nach einer Standard-Methode (T.K. Kirk, s. o.), die auf der Oxidation von Veratrylalkohol zum Aldehyd basiert. Angaben in Units/l (Oxidation von 1 µmol Veratrylalkohol zum Aldehyd/min, Extinktionszunahme bei 308 nm).

Die Enzyme wurden durch Ultrafiltration (Ausschlußgrenze 10 kDa) aus dem Kulturmedium gewonnen, gegen destilliertes Wasser dialysiert und anschließend lyophilisiert.

Die Fraktionierung bzw. Reinigung der Enzymrohpräparate im präparativen Maßstab wird durch Flüssigkeitschromatographie an einer Anionenaustauscher-Matrix (Q-Sepharose der Fa. Pharmacia) vorgenommen. Die Eluierung der einzelnen Fraktionen von der Säule erfolgt mit einem linearen Gradienten von Na-Acetat-Puffer (10 mM bis 600 mM) mit dem pH-Wert 6.

Als weitere Weißfäulepilze für die Gewinnung von Ligninasen gemäß der Erfindung kommen z. B. Stämme von Coriolus, Schizophyllum, Phlebia, Ganoderma, Fomes usw. in Betracht.

## Ansprüche

1. Verfahren zur Gewinnung ligninolytischer Enzyme durch Kultivierung von Weißfäulepilzen,
**dadurch gekennzeichnet,**
daß der Pilz-Kultur bei beginnender Verfärbung zumindest 0,1 g/l Mangandioxid-Pulver zugesetzt und der pH-Wert im Medium während der gesamten Kulturdauer zwischen pH 4,8 und 5,0 konstant gehalten wird.

2. Verfahren nach Anspruch 1,
**dadurch gekennzeichnet,**
daß das Mangandioxid als feinteiliges Pulver mit einer Korngröße < 10 µm zugesetzt wird.

3. Verfahren nach Anspruch 1 oder 2,
**dadurch gekennzeichnet,**
daß das Mangandioxid in Mengen von 0,3 bis 5 g/l und insbesondere um 1 g/l Kulturmedium zugesetzt wird.

4. Verfahren nach einem der vorangehenden Ansprüche,
**dadurch gekennzeichnet,**
daß das Mangandioxid einer Kultur mit auf einem Träger aufgewachsenen Pilzen zugesetzt wird.

5. Verfahren nach einem der vorangehenden Ansprüche,
**dadurch gekennzeichnet,**

3

daß die Enzyme vom Kulturmedium wiederkehrend durch Ultrafiltration abgesondert und das Restkultursubstrat recycliert wird.

6. Verfahren nach einem der vorangehenden Ansprüche,

**dadurch gekennzeichnet,**

daß als Weißfäulepilz ein Stamm von Phanerochaete chrysosporium kultiviert wird.